# EUROPEAN PATENT SPECIFICATION

(11) **EP 0 712 297 B1**
(45) Date of publication and mention of the grant of the patent: **17.03.2004**
(21) Application number: 94925119.3
(22) Date of filing: 28.07.1994
(51) Int. Cl.: A61B 19/00

(54) **SEGMENTED PLIABLE INTRASTROMAL CORNEAL INSERT**
ZUSAMMENLEGBARER, INTRASTROMALER, KORNEALER EINSATZ AUS MEHREREN SEGMENTEN
INSERT SOUPLE ET SEGMENTE DESTINE AU STROMA CORNEEN

(30) Priority: 02.08.1993 US 101440
(43) Date of publication of application: 22.05.1996
(60) Divisional application: 03029374.0
(73) Proprietor: Addition Technology, Inc, Des Plaines, Illinois, (US)
(72) Inventor: SILVESTRINI, Thomas, A., Alamo, CA 94507 (US)
(74) Representative: Goldin, Douglas Michael
(86) International application number: PCT/US1994/008458
(87) International publication number: WO 1995/003747

(56) References cited:
- WO-A-93/13724
- WO-A-94/03129
- US-A- 4 936 825
- US-A- 4 961 744
- US-A- 5 098 443
- US-A- 5 290 271
- US-A- 5 372 580
- DATABASE WPI Section PQ, Week 9345 Derwent Publications Ltd., London, GB; Class P, AN 93-358291 XP002016451 & SU-A-1 771 730 (ALMA-ATA DOCTORS TRAINING INST.)
- VESTNIK OFTALMOLOGII, vol. 3, 1991, pages 23-31, XP002022279 N.E. TEMIROV ET AL.: "REFRACTION RING-LIKE TUNNEL KERATOPLASTY FOR THE CORRECTION OF HIGH MYOPIA"

## Description

This invention is a pliable intrastromal corneal insert designed to be inserted into an interlamellar channel made within the cornea of a mammalian eye. It is made of a physiologically compatible polymer and may be used to adjust corneal curvature and thereby correct vision abnormalities. The insert or segment may also be used to deliver therapeutic or diagnostic agents to the corneal interior or to the interior of the eye. The insert subtends at least a portion of a ring, or "arc", encircling the anterior cornea outside of the cornea's field of view but within the cornea's frontal diameter, but may be used in multiples to form complete arcs or to form constructs of varying thickness.

Anomalies in the overall shape of the eye can cause visual disorders. Hyperopia ("farsightedness") occurs when the front-to-back distance in the eyeball is too short. In such a case, parallel rays originating greater than 20 feet from the eye focus behind the retina. In contrast, when the front-to-back distance of eyeball is too long, myopia ("nearsightedness") occurs and the focus of parallel rays entering the eye occurs in front of the retina. Astigmatism is a condition which occurs when the parallel rays of light do not focus to a single point within the eye, but rather have a variable focus due to the fact that the cornea refracts light in a different meridian at different distances. Some degree of astigmatism is normal, but where it is pronounced, the astigmatism must be corrected.

Hyperopia, myopia, and astigmatism are usually corrected by glasses or contact lenses. Surgical methods for the correction of such disorders are known. Such methods include radial keratotomy (see, e.g., U.S. Patents Nos. 4,815,463 and 4,688,570) and laser corneal ablation (see, e.g., U.S. Patent No. 4,941,093).

Another method for correcting those disorders is through the implantation of polymeric rings (intrastromal corneal rings or "ICR's") in the eye's corneal stroma to change the curvature of the cornea. Previous work involving the implantation of polymethylmethacrylate (PMMA) rings, allograft corneal tissue, and hydrogels is well documented. One of the ring devices involves a split ring design which is inserted into a channel previously dissected in the stromal layer of the cornea. A minimally invasive incision is used both for producing the channel and for inserting the implant. See, for instance, the use of PMMA intrastromal rings in U.S. Patents Nos. 4,452,235 to Reynolds; 4,671,276 to Reynolds; 4,766,895 to Reynolds; and 4,961,744 to Kilmer et al. These documents suggest only the use of ICR's which completely encircle the cornea.

The use of soft polymers as intrastromal inserts is not widely known. For instance, U.S. Patent No. 5,090,955 to Simon, suggests an ICR which is made by introducing a settable polymer or gel into an intrastromal channel which has been previously made and allowing the polymer to set. This procedure does not allow the surgeon to specify the precise size of the resulting ring nor is it a process which allows precise control of the pathway of the flowing polymer within the eye since the gel must simply conform to the shape of the intrastromal channel. However, it does show the concept of using arcuate channels containing a gel-based insert centered about the corneal.

Temirov et al, "Refractive circular tunnel keroplasty in the correction of high myopia", Vestnik Oftalmologii 1991: 3-21-31, suggests the use of collagen thread as ICR material.

These publications do not suggest the introduction of pliable polymeric inserts into the cornea for the correction of various visual aberrations. The publications do not imply that the devices may be used to introduce therapeutic or diagnostic materials into the corneal intrastromal space.

US-A-5 098 443 describes a series of implants for intraocular and/or intraorbital use. The implants provide for the controlled release of pharmacological agents. The implants are substantially C-shaped rings and are inserted through incisions made in the eye wall or are sutured around the globe of the eye. The C-shaped rings may be formed from biodegradable polymers so as to release a drug as the polymer biodegrades or the implant may be in the form of a hollow flexible polymeric cocoon with the drug disposed therein for slow release by osmosis.

According to the present invention there is provided an intrastromal corneal insert adapted for introduction into the cornea of a human eye, the insert comprising a pliable, physiologically compatible, pliable, synthetic, polymeric segment adapted to extend along a circumferentially extending intrastromal intracomeal channel in said eye and subtend an arc less than 360° of the cornea's circumference when inserted into said intrastromal channel, said segment, alone or in combination with one or more of said segments so inserted, being adapted to effect refractive correction.

Multiple different embodiments of preferred intrastromal corneal insert will be described hereinafter. In each case, the insert is of a selected size and composition and will, after insertion into the interlamellar channel, conform to the shape of the channel and alter the anterior shape of the cornea. It need not conform to the intrastromal channel shape prior to insertion. An insert may be used in isolation, to encircle a portion of the cornea. Alternatively inserts may be used in isolated multiples, in cooperative multiples, or as segments in a larger assemblage encircling at least a portion of the cornea. The insert may be of one or more synthetic polymers, hydrophilic or hydrophobic, or may be a hybrid device comprising layered materials. Optionally, the insert may contain filamentary material in the form of a single or multiple threads, random included filaments, or woven mattes to reinforce the insert during, e.g., insertion or removal from the intrastromal channel.

The insert may be hollow and may be filled with a biologic agent, drug or other liquid, emulsified, or time-release eye treatment or diagnostic material. The insert may contain a gel, viscous, or visco-elastic material which remains in such a state after introduction.

When a hybrid, the inner portion may comprise variously a composite of low modulus polymers or a single low modulus polymer. The inner portion may also comprise a polymeric material which is polymerized in situ after introduction into the hollow center layer. The inserts may be trimmed from a larger or longer insert prior to or after insertion into the eye. The larger precursor may be of a constant size or diameter or may be of a variable size or diameter. The variable size or diameter insert precursor allows the surgeon to select a size which will make the necessary visual correction. The long precursor may then be clipped and removed.

These inventive segmented inserts may be introduced into the corneal stroma using techniques involving the steps of providing an intrastromal channel which traverses at least a portion of the circumcorneal rotation. Specific indications, such as astigmatism, may be rectified by insertion of one or more of the inserts into a partial intrastromal channel to flatten the steeper portions of the anterior corneal surface without insertion of a complete intracorneal ring (ICR).

If hydratable polymers are used, they may be hydrated before or after introduction into the intrastromal passageway created by the surgical device used to introduce these devices into the eye. If the outer layer is hydrated before insertion into the eye, the final size of the insert is set before that insertion. If the hydratable polymers are allowed to hydrate within the corneal space, the device (if appropriate polymers are chosen) will swell within the eye to its final size. If prehydrated, the outer layer often provides a measure of lubricity to the device, allowing it to be inserted with greater ease. Other of the noted low modulus polymers may also provide such lubricity.

Figure 1 is a schematic illustration of a horizontal section of the eye.

Figure 2 is a schematic illustration of the anterior portion of the eye showing the various layers of the cornea.

Figures 3A and 3B show respectively a front view and a cross section of a typical intracorneal insert made according to the invention.

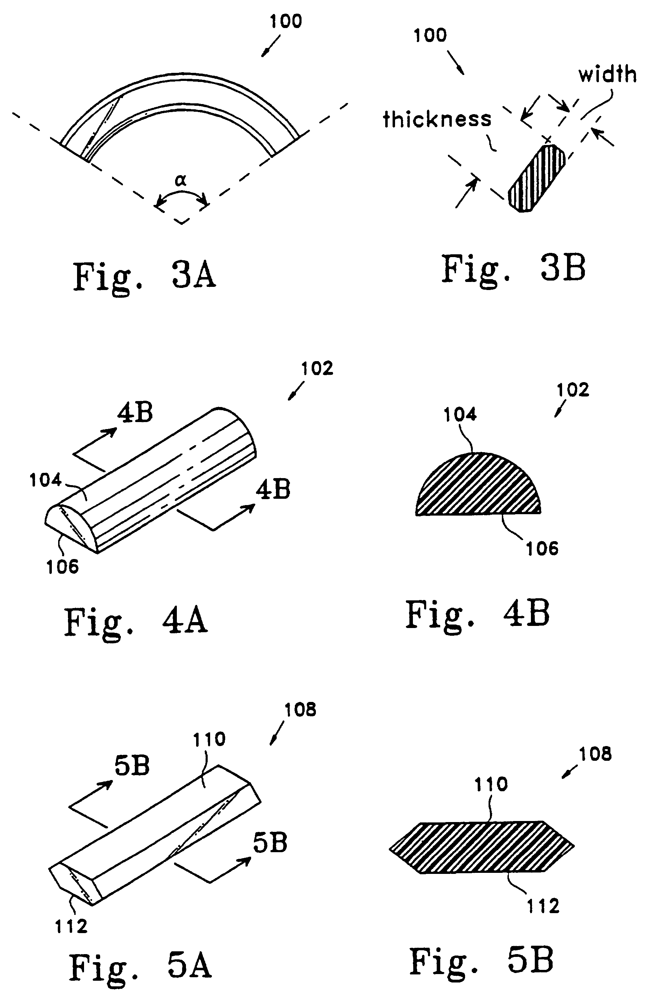

Each of Figures 4A and 4B, 5A and 5B, 6A and 6B, and 7A and 7B, shows respectively a front view ("A" drawing) and a cross section ("B" drawing) of various intracorneal inserts made according to the invention.

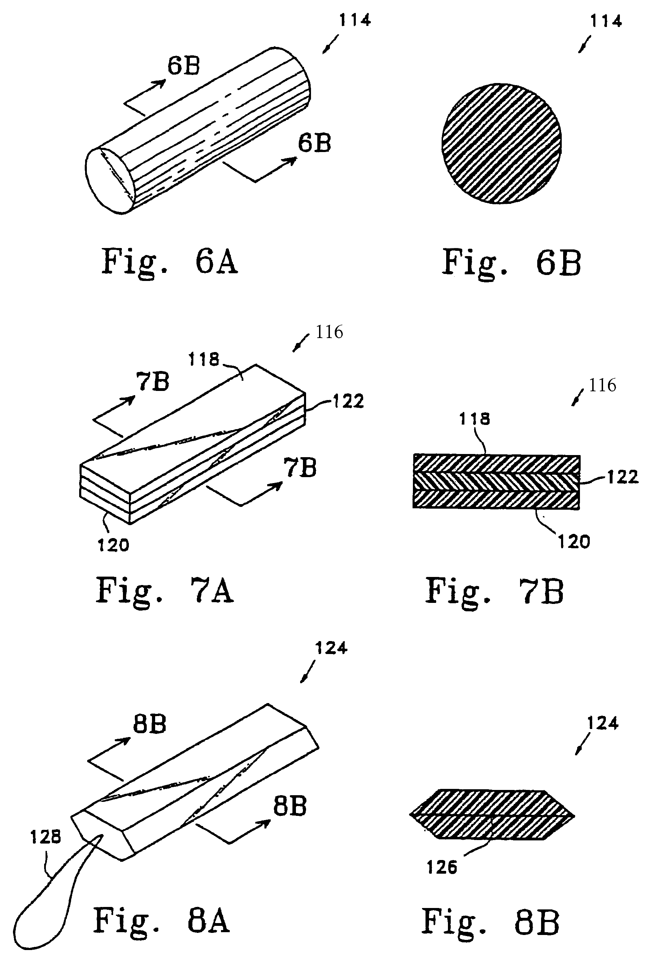

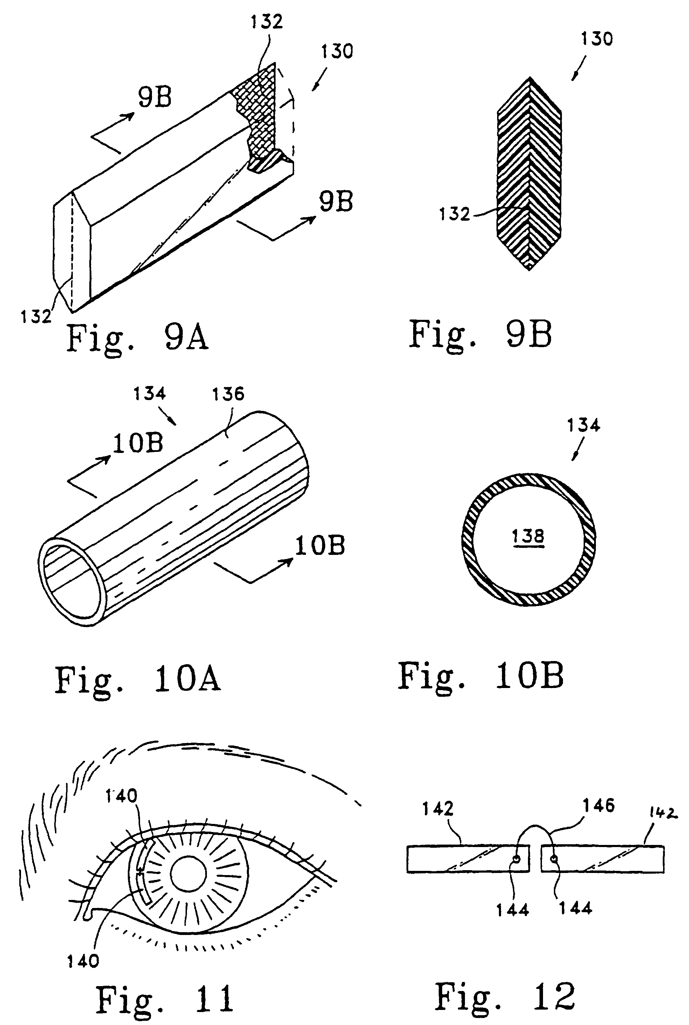

Each of Figures 8A and 8B and 9A and 9B shows respectively a front view ("A" drawing) and a cross section ("B" drawing) of various intracorneal inserts having filamentary reinforcing made according to the invention.

Figures 10A and 10B show respectively a front view and a cross section of a soft, filled intracorneal insert made according to the invention.

Figure 11 depicts a front view of an end-to-end assemblage of intracorneal segments placed within a human cornea.

Figure 12 shows an assemblage of intrastromal segments joined with a clasp.

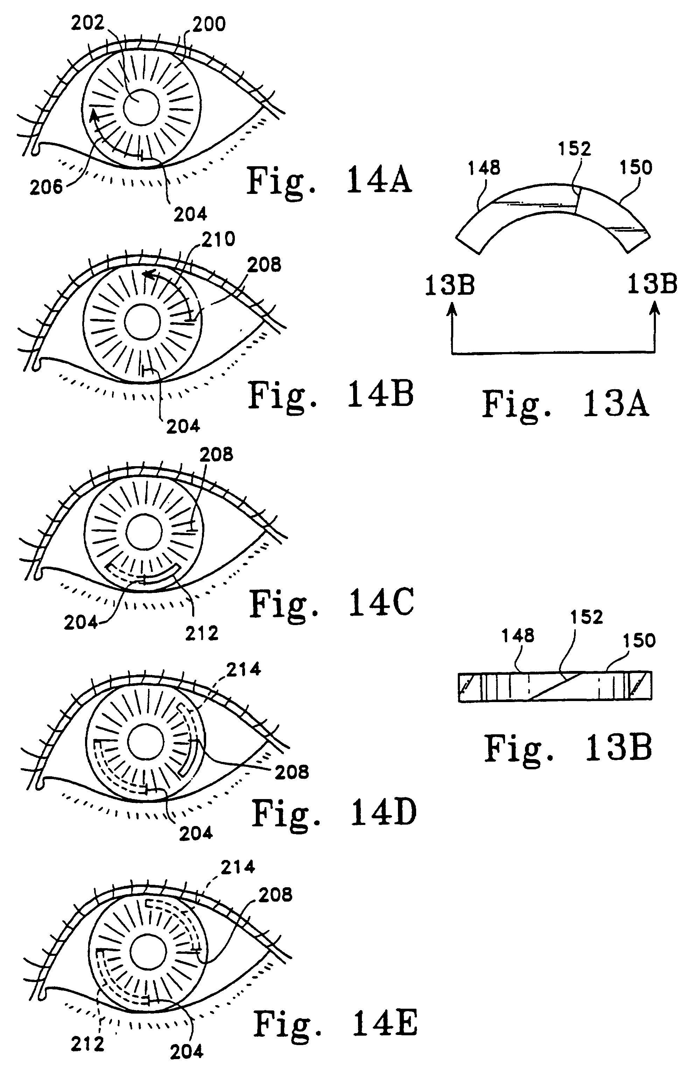

Figures 13A and 13B show top and side views of overlapping segments.

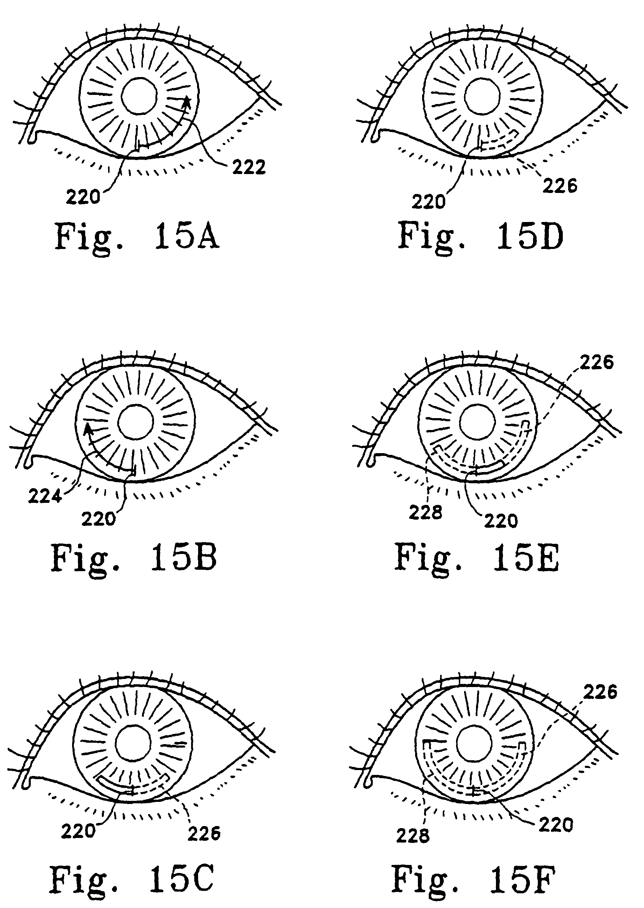

Figures 14A-14E and 15A-15F show schematic processes for introducing multiple inserts into the human cornea.

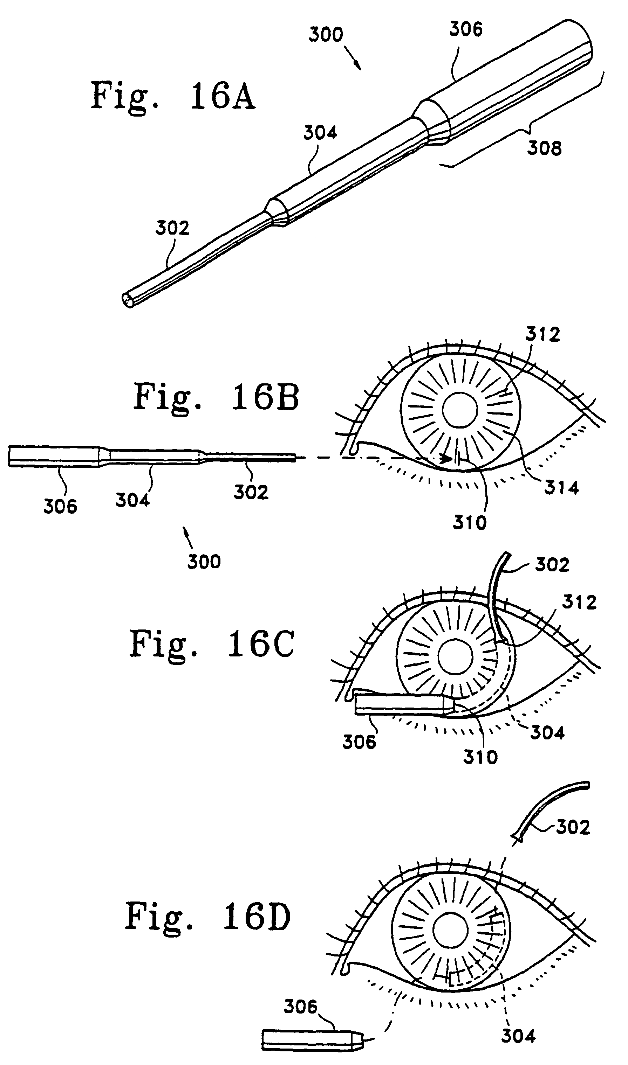

Figure 16A shows an insert precursor having a variety of diameters. Figures 16B-D schematically show a procedure for use of the insert precursor shown in Fig. 16A.

Prior to explaining the details of the inventive devices, a short explanation of the physiology of the eye is needed to appreciate the functional relationship of these intracorneal inserts or segments to the eye.

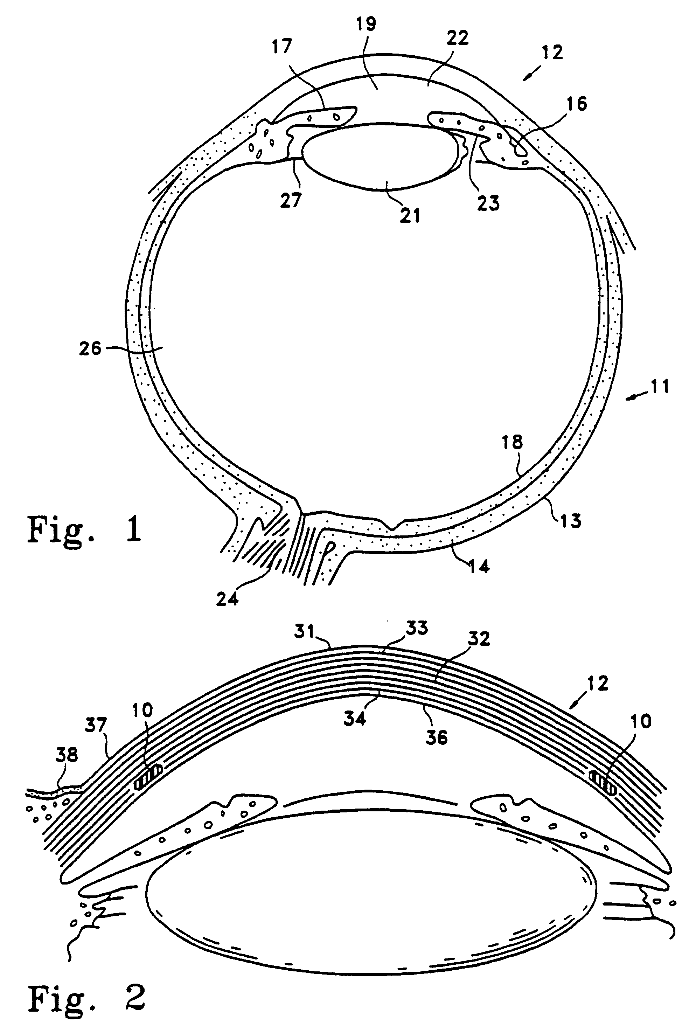

Figure 1 shows a horizontal cross-section of the eye with the globe (11) of the eye resembling a sphere with an anterior bulged spherical portion representing the cornea (12).

The globe (11) of the eye consists of three concentric coverings enclosing the various transparent media through which the light must pass before reaching the light-sensitive retina (18). The outermost covering is a fibrous protective portion the posterior five-sixths of which is white and opaque and called the sclera (13), and sometimes referred to as the white of the eye where visible to the front. The anterior one-sixth of this outer layer is the transparent cornea (12).

A middle covering is mainly vascular and nutritive in function and is made up of the choroid, ciliary body (16), and iris (17). The choroid generally functions to maintain the retina (18). The ciliary body (16) is involved in suspending the lens (21) and accommodation of the lens. The iris (17) is the most anterior portion of the middle covering of the eye and is arranged in a frontal plane. It is a thin circular disc similar in function to the diaphragm of a camera, and is perforate near its center by a circular aperture called the pupil (19). The size of the pupil varies to regulate the amount of light which reaches the retina (18). It contracts also to accommodation, which serves to sharpen the focus by diminishing spherical aberration. The iris divides the space between the cornea (12) and the lens (21) into an anterior chamber (22) and the posterior chamber (23). The innermost portion of covering is the retina (18), consisting of nerve elements which form the true receptive portion for visual impressions.

The retina (18) is a part of the brain arising as an outgrowth from the fore-brain, with the optic nerve (24) serving as a fiber tract connecting the retina part of the brain with the fore-brain. A layer of rods and cones, lying just beneath a pigmented epithelium on the anterior wall of the retina serve as visual cells or photoreceptors which transform physical energy (light) into nerve impulses.

The vitreous body (26) is a transparent gelatinous mass which fills the posterior four-fifths of the globe (11). At its sides it supports the ciliary body (16) and the retina (18). A frontal saucer-shaped depression houses the lens.

The lens (21) of the eye is a transparent biconvex body of crystalline appearance placed between the iris (17) and vitreous body (26). Its axial diameter varies markedly with accommodation. A ciliary zonule (27), consisting of transparent fibers passing between the ciliary body (16) and lens (121 serves to hold the lens (21) in position and enables the ciliary muscle to act on it.

Referring again to the cornea (12), this outermost fibrous transparent coating resembles a watch glass. Its curvature is somewhat greater than the rest of the globe and is ideally spherical in nature. However, often it is more curved in one meridian than another giving rise to astigmatism. A central third of the cornea is called the optical zone with a slight flattening taking place outwardly thereof as the cornea thickens towards its periphery. Most of the refraction of the eye takes place through the cornea.

Figure 2 is a more detailed drawing of the anterior portion of the globe showing the various layers of the cornea (12) making up the epithelium (31). Epithelial cells on the surface thereof function to maintain transparency of the cornea (12). These epithelial cells are rich in glycogen, enzymes and acetylcholine and their activity regulates the corneal corpuscles and controls the transport of water and electrolytes through the lamellae of the stroma (32) of the cornea (12).

An anterior limiting lamella (33), referred to as Bowman's membrane or layer, is positioned between the epithelium (31) and the stroma (32) of the cornea. The corneal stroma (32) are made up of lamellae having bands of fibrils parallel to each other and crossing the whole of the cornea. While most of the fibrous bands are parallel to the surface, some are oblique, especially anteriorly. A posterior limiting lamella (34) is referred to as Descemet's membrane. It is a strong membrane sharply defined from the stroma (32) and resistant to pathological processes of the cornea.

The endothelium (36) is the most posterior layer of the cornea and consists of a single layer of cells. The limbus (37) is the transition zone between the conjunctiva (38) and sclera on the one hand and the cornea (12) on the other.

Figure 3A shows a front view of one variation of an insert made according to the invention and Figure 3B shows a cross section of that insert. These segments are suitable for insertion into the appropriately prepared interlamellar, intrastromal, intracorneal channel. Generally, the intrastromal segment is installed into the eye in the following manner: A small radial incision is made at the corneal radius at which the intrastromal segment is ultimately to be installed about the cornea. A dissector in the form of a split ring having a point suitable for producing the interlamellar channel in the corneal stroma is introduced into the stromal space through the small incision. The dissector is then rotated in such a fashion that a generally semicircular or arc-shaped channel is formed at least partially circling the cornea at the chosen radius. After a channel of the proper length is achieved, the cutting step is concluded. The dissector is then rotated in the opposite direction to withdraw it from the tunnel or channel thus formed. As is explained in more detail below, a pliable intrastromal segment is then introduced into the channel.

As is shown in Figure 3A, the segment (100), after it is inserted into the eye, conforms to the shape of the intrastromal channel subtending some specific portion of the circumference of the cornea equal to a value of "α", which value is less than 360°, but preferably less than 320°, most preferably less than 270°. I refer to this angle as the "arc angle". The value of "α" is dependent upon the indication to be resolved and the physical arrangement of the sector (or sectors) as they are installed in the eye. For instance, often the value of "α" is 20 to 90° for the correction of modest astigmatic aberrations resulting in astigmatism. Similarly, if the segments are used in conjunction with each other such as is described below, the value of "α" may be any of a wide range of values. In any event, for definitional purposes, the opposite ends of a single "segment" do not meet when the segment is inserted into an intrastromal channel. However, as described below, the ends of a segment may overlap with the end of another segment, may abut another segment, or may be parallel with another segment when they are placed in an intrastromal channel.

Figure 3B shows the cross section of the sector. For convenience, the chosen conventions for thickness and width are shown on Figure 3B. The typical width is often between 0.127 mm (0.005 inches) and 6.35 mm (0.250 inches). The typical thickness is often between 0.127 mm (0.005 inches) and 2.03 mm (0.080 inches). Both of these parameters (along with certain other variables such as the cross-sectional shape of the device and its constituent polymers) determine, in large part, the level of correction achievable by use of the insert.

The devices of this invention are pliable. By "pliable", I mean that the device, prior to its insertion into the eye, is quite flexible and desirably is not preformed to the shape of the intrastromal channel noted above. Specifically, and most desirable, "pliable" means that the device is transformed from a previous shape (that prior to insertion into the intrastromal channel) into the shape of the intrastromal channel only by imposition of the force inherently found in the channel walls.

The materials used in these inserts may be physiologically acceptable, low modulus polymers, e.g., those having a modulus of elasticity below about 24.1 MPa (3.5 kpsi), more preferably between 6.9 kPa and 6.9 MPa (1 psi and 1 kpsi), and most preferably between 6.9 kPa and 3.44 MPa (1 psi and 500 psi), which are physiologically compatible with the eye. Most polymeric materials used in soft contact lenses are suitable the segments of this invention. The class includes physiologically compatible elastomers and such crosslinked polymeric gels as polyhydroxyethylmethacrylate (Poly-HEMA) or polyvinylpyrrolidone (PVP), polyethylene oxide, or polyacrylates, polyacrylic acid and its derivatives, their copolymers and interpolymers, and the like as well as biologic polymers such as crosslinked dextran, crosslinked heparin or hyaluronic acid.

In many instances, the intrastromal segments may be hybrid, that is to say, the segments are made up of a number of polymeric layers often with a soft or hydratable polymer on their outer surface. These hybrid segments will be described with greater particularity below. Partially hydrated or fully hydrated hydrophilic polymers are typically slippery and consequently may contribute to the ease with which the insert may be introduced into the interlamellar tunnel. It is usually desirable to (at least partially) hydrate the hybrid intrastromal segment in that, otherwise, the intrastromal segment during its traverse through the tunnel may desiccate the path and begin to stick to the interior wall of the tunnel.

The intrastromal segments may be lubricated with suitable ocular lubricants such as hyaluronic acid, methylethyl cellulose, dextran solutions, glycerine solutions, polysaccharides, or oligosaccharides upon its introduction to help with the insertion particularly if one wishes to insert intrastromal segments of hydrophilic polymers without prior hydration. If a hybrid segment having a hydrophilic polymeric covering or a segment comprising a hydrophilic polymer is inserted into the eye without prior hydration, subsequent to the insertion, the intrastromal segment will swell to its final size or thickness within the eye. This swelling often permits the inclusion of larger intrastromal segments than would normally be accommodated within normal sized intrastromal channels.

Low modulus polymers used in this invention are often absorbent, particularly if they are hydratable, and may be infused with a drug or biologic agent which may be slowly released from the device after implantation of the intrastromal segment. For instance, the low modulus polymer may be loaded with a drug such as dexamethasone to reduce acute inflammatory response to implanting the device. This drug helps to prevent undesirable scarring or vascular ingrowth toward the intrastromal segment. Similarly, heparin, corticosteroids, antimitotics, antifibrotics, antiinflammatories, anti-scar-forming, anti-adhesion, and antiangiogenesis factors (such as nicotine adenine dinucleotide (NAD⁺)) may be included to reduce or prevent angiogenesis and inflammation.

Clearly, there are a variety of other drugs suitable for inclusion in the intrastromal segment. The choice will depend upon the use to which the drugs are put.

Each of Figures 4A and 4B, 5A and 5B, 6A and 6B, and 7A and 7B show respectively a front view ("A" drawing) and a cross section ("B" drawing) of various intracorneal inserts suitable for use in the inventive method.

Figure 4A shows a front view of an intracorneal insert (102). Viewed in cross section in Figure 4B, the generally smooth convex front surface (104) and planar rear surface (106) may be seen.

Figure 5A shows a front view of an intracorneal insert (108). As with the devices shown in Figures 4A and 4B, the intracorneal inserts may taper either or both in width and in thickness or may have blunt, non-trimmed ends. Viewed in cross section in Figure 5B, the generally hexagonal shape may be seen. The generally planar front surface (110) and planar rear surface (112) may be seen. Our previous experience with IntraCorneal Rings ("ICRs") has demonstrated that the use of such a shape in the cornea is generally less traumatic than one of a rectangular cross section and yet, because of the similarity of the shape to that of the intrastromal formed by the blade producing the channel, is often considered to be the maximum cross sectional volume achievable in such configuration.

Figure 6A shows a front view of an intracorneal insert (114). Figure 6B shows the generally round cross section. The cross section may also be oval-shaped with the major axis of the oval either as the width or the thickness or neither.

Figure 7A shows a front view of a hybrid intracorneal insert (116). Viewed in cross section in Figure 7B, the generally parallelepipedal shape may be seen. This set of Figures is to show the concept of a multilayered insert made up of polymers of different characteristics. In this example of a multi-layered insert, the hybrid intrastromal segment has inner (118) and outer faces (120) of polymers having low moduli of elasticity. Low modulus polymers are those having a modulus of elasticity below about 24.1 MPa (3.5 kpsi), more preferably between 6.9 kPa and 6.9 MPa (1 psi and 1 kpsi), and most preferably between 6.9 kPa and 3.44 MPa (1 psi and 500 psi). They must be physiologically compatible with the eye. As was noted above, this class of polymers includes most polymeric materials used in soft contact lenses.

The inner portion or core (122) as shown in Figure 7B may also be a physiologically compatible polymer having a low modulus of elasticity.

If hydratable polymers are chosen for the outside layers, the extent to which those outer layers swell upon hydration is dependent upon the type of polymer chosen and, when the polymer is hydratable, upon the amount of cross-linking found in the outer layers (118) and (120), and upon the thickness of the layer. Generally speaking, the more highly linked the hydratable polymer, the smaller the amount of volume change upon hydration. Conversely, a polymer having only sufficient cross-linking for strength in the service in which this device is placed, will have a somewhat lower level of cross-linking. Alternatively, a substantially nonswellable polymer system may be formed of a hydrogel physically interpenetrated by another polymer which does not hydrate. Suitable hydrophilic polymers include polyhydroxyethylmethacrylate (pHEMA), N-substituted acrylamides, polyvinyl pyrrolidone (PVP), polyacrylamide, polyglyceryl methacrylate, polyethylene oxide, polyvinyl alcohol, polyacrylic acid, polymethacrylic acid, poly(N,N-dimethylaminopropyl-N'-acrylamide) and their copolymers and their combinations with hydrophilic and hydrophobic comonomers, crosslinks, and other modifiers. Thermoplastic hydrogels include hydropolyacrylonitrile, polyvinyl alcohol derivatives, hydrophilic polyurethanes, styrene-PVP block copolymers and the like.

The thickness of the outer layer depends in large function upon the intended use of the intrastromal segment. If the outer layer is used to provide a swellable outer layer which does not add significantly to the size of the intrastromal segment or is used functionally as a lubricant layer, the other layer may be quite thin even to the point of a layer of minimum coverage, perhaps as thin as a single molecule.

Of course, the inner and outer layers may be multiple layers of low modulus polymers including an outer hydrophilic polymer layer and an inner hydrophobic polymer; a variety of hydrophilic polymers; etc.

Additionally, the inventive device shown in Figures 7A and 7B need not have inner (118) and outer (120) layers over the entire intrastromal segment. For instance, to alleviate astigmatism, an intrastromal segment having a thicker portion and a substantially thinner portion may be desired. An intrastromal segment having an inner core of a low modulus polymer and an outer covering of a swellable polymer might be chosen. The eye surgeon would remove a portion of the intrastromal segment's exterior coating or face prior to introducing the intrastromal segment into the eye. Further, and as will be discussed below in greater detail, hydrophilic polymers are more easily infused with therapeutic and diagnostic materials than are the high modulus materials. In the variation just noted, the insert may then be used to deliver the infused therapeutic and diagnostic materials in a greatly delimited treatment or diagnostic area.

Figure 8A shows a front view of an intracorneal insert (124). In this variation, the insert includes at least one flexible, longitudinal, filamentary inclusion to provide a measure of axial strength during the insertion procedure. The filamentary inclusion (126) may be seen in cross section in Figure 8B. A generic cross-sectional shape is depicted in Figure 8B. The filamentary inclusion (126) is shown with a loop (128) for attachment to an insertion tool. The loop (128) is optional and may be omitted depending upon the manner in which the insert is to be inserted. The filamentary inclusion (128) may be produced from a wide variety of bio-compatible materials including Kevlar (RTM), Dacron (RTM), etc. Additionally, the filamentary material may be placed within the insert in a random fashion.

Figure 9A shows a partial cross-section, front view of an intracorneal insert (130). In this variation, the insert includes a flexible, fabric inclusion (132) to provide axial strength during the insertion procedure. The inclusion (132) may be seen in cross-section in Figure 9B. A generic cross-sectional shape is depicted in Figure 9B. The fabric inclusion (132) may also be produced from a wide variety of bio-compatible materials including Kevlar, Dacron, etc. A loop (not shown) as shown for the variation found in Figure 8A and 8B,may also be included for ease of insertion if so desired.

The variations shown in Figures 8A, 8B, 9A, and 9B may be of virtually any cross-section which will conform to the shape of the intrastromal channel upon insertion. Methods of binding the low modulus polymeric coating to the filamentary or fabric inclusion are well known and need not be described in detail here. Such methods include molding the filament or fabric in place or using an adhesive or intermediate polymeric tie-layer to assure contact and adherence of the fabric to the outer polymeric layer or layers.

Figure 10A is a front quarter view of a variation of the intrastromal segment (134) made of a low modulus polymer system hydratable outer coating (136). Figure 10B shows the inner cavity (138). This intrastromal segment may be inserted into the intrastromal space created by the dissector as a covering on a tool similar to the dissector which created the intracorneal channel. Once in position the insertion tool is rotated out of the intrastromal segment leaving the shell within the stroma.

Figure 10 shows the inner cavity (138) which may be filled with a biologic, a drug or other liquid, or biologically active eye treatment material. These devices may be tied or pinched or crimped or otherwise connected at their point of insertion by known techniques.

The shell (136) may be injected with a gel or with a settable soft polymer core, allowed to expand to a desired thickness, and set. Polymeric gels which do not polymerize in situ are preferred. Suitable injectable polymers are well known but include polyHEMA hydrogel, cross-linked collagen, cross-linked hyaluronic acid, siloxane gels, and organic-siloxane gels such as cross-linked methyl vinyl siloxane gels.

Figure 11 shows a variation of the invention in which an assemblage of the inventive intrastromal segments (140) are formed into a polymeric ring or, at least, into an assemblage within the intracorneal space. The two segments (140) depicted in Figure 11 may be of any of the individual variations shown in the Figures above and need not be connected in any way. The segments may be of similar or quite different configurations depending upon the indication to be remedied. Additionally, they may be inserted in separately produced intrastromal channels which may, or may not, be in communication within the cornea. Such individual insertion will be discussed in more detail below.

Figure 12 shows a similar assemblage in which the intracorneal segments (142) are held together using open holes (144) and a clip (146) which may be a simple wire or other suitable joining device. An assemblage such as is seen in Figure 12 may be advantageously inserted from a single central opening, as will be described below.

Figures 13A and 13B show a variation of the inventive intracorneal inserts in which two or more inserts overlap to form an assemblage. The top view shown in Figure 13A depicts the assemblage of segment (148) and segment (150) meeting at junction (152) as found in the eye. The assemblage need not be formed of segments of the same or similar width or thickness or material of construction nor need the assemblage be limited to the semicircle shown in Figure 13A. Although a front-to-back assemblage is depicted in Figure 13B, the junction (150) between the sections (148 & 150) may be of any other design which is allows contact between the adjoining sections and remains relatively immobile after the placement in the cornea. The intrastromal channel normally exerts significant force against the assemblage and will maintain the sectors in the depicted relational position within the eye.

The ends of the inserts may be substantially overlapped so to form a thick insert for the overlapping area so to correct an astigmatic problem. The inserts may completely overlap within a channel. The two inserts may be of differing crop sections or diameters. Further, rather than overlapping, the inserts may actually be stacked one on top of the other.

Figures 14A-14E schematically portray a method for the insertion of the segments described above in which partial arc segments are introduced into separate sections of the corneal circumference outside of the "sight" area of that cornea.

In Figure 14A, the frontal shows the iris (200) and the pupil (202), As was described above, the cornea is clear and is not visible in these drawings. Insertion of the inventive device is a reasonably simple surgical procedure. An entry slit (204) is made radially into the cornea. A dissector blade is introduced into the entry slit (204) and turned in the direction of the arrow (206) to form a partial intrastromal channel of a desired length. As is then shown in Figure 14B, second entry slit (208) may then be made in the cornea and a second intrastromal channel be made in the direction of the arrow (210).

Figure 14C shows the introduction of the first inventive segment (212) into the first entry slit (208). Figure 14D shows the first segment (212) in its final resting position and the introduction of the second segment (214) into the second entry slit (208). Finally Figure 14E shows both first segment (212) and second segment (214) in their final position within the cornea. This demonstrates the flexibility of the procedure in that either left or right "hand" insertion is appropriate and the intrastromal channel need ont be a complete circle about the cornea. Further, it should be noted that the first segment (212) and second segment (214) may be of differing diameters or of differing arc lengths depending upon the indication to be resolved.

Figures 15A-15F schematically portray a method for the insertion of the segments described above in which partial arc segments are introduced into separate sections of the corneal circumference outside of the "sight" area of that cornea through a single entry slit.

Figure 15A shows the making of the initial entry slit (220) radially into the cornea. A dissector blade is introduced into the entry slit (220) and turned in the direction of the arrow (222) to form a partial intrastromal channel of a desired length. As is shown in Figure 15B, a second intrastromal channel is made in the direction of the arrow (224) from the same entry slit (222).

Figure 15C shows the introduction of the first segment (226) into the entry slit (222). Figure 15D shows the first segment (226) in its final resting position. Figure 15E shows the introduction of the second segment (228) into the entry slit (220). Finally Figure 15F shows both first segment (226) and second segment (228) in their final position within the cornea.

Because of the nature of these pliable inserts, a large measure of adaptability is available in the process of inserting the devices. For instance, I have found that when using various pliable inserts (particularly with ocular lubricants) that the inserts may be "pushed" nearly 180° around a previously created intrastromal channel for insertion and then easily removed, if so desired. This observation means that the following procedure may be used. The eye of a person having myopia and/or astigmatism may be measured to determine the proper amount of correction needed. From this information, the size and placement of one or more segments may then be chosen. For instance, the selected sections might be two inserts of 30° arc angle and 2.54 mm x 2.54 mm (100 mils x 100 mils) cross-section at two opposing meridians. After insertion in the appropriate channels, the vision of the eye might again be measured. If insufficient correction of an indication is found, the insert may be withdrawn and a larger size selected and inserted. If an astigmatic aberration is introduced, the insert may be withdrawn (partially or completely) and trimmed prior to complete re-insertion. Such adjustability is not normally available when dealing with gel-based rings or with surgical techniques based on radial keratotomy.

Figure 16A shows a pliable insert precursor, which may be trimmed prior to or after insertion into the intrastromal channel. In this instance, the insert precursor (300) is made up of a number of sections -- three are illustrated in Figure 16A although such is not necessary -- of differing size cross-sections, which cross-sections increase in steps along the axis of the insert precursor. The relative cross-sectional area of small section (302) is smaller than that of the midsection (304) which, in turn, is smaller than that of the large section (306). A section (308) is of any convenient length but normally need not be much longer than the length of a intrastromal channel formed within the circumference of the cornea. It may be of a shorter length if so desired.

Figures 16B-16D show one method for introduction of the precursor insert into a partial intrastromal channel forming an arc of about 120°. In this illustrative example, two small radial incisions (310 and 312) have been made and an intrastromal channel (314) made between the two incisions (310 and 312). The insert precursor (300) is introduced into one of the incisions (310) and, as is shown in Figure 16C, out of the other incisions (312). In this example the proper amount of astigmatic correction was provided by the mid-section (304) of the insert precursor (300), and so the precursor was slid through the intrastromal channel until the small section (312) protruded out of the incision (312). Figure 16D shows that the protruding small section (302) and large section (306) are snipped, leaving the mid-section (304) in the intrastromal channel. The incisions (310 and 312) may be sutured shut.

This arrangement, as is shown in Figures 16B-16D, might be used to correct an astigmatism. However, the procedure could just as easily be used within an intrastromal channel which completely encircles the cornea.

The inserts may be useful in the treatment of astigmatism, myopia, or the combination of the two. In each case, segments of differing arc length are preferred. For the treatment of astigmatism where no myopic correction is needed, segments of between about 20° and 90°, preferably between about 20° and 60° may be used. Where treatment of astigmatism and myopia is required, segments of between about 45° and 160°, preferably between about 60° and 90° may be used. Where the treatment of myopia where no astigmatic enhancement is required, segments of between about 90° and 360°, preferably between about 90° and 270° may be used.

The terms and expressions which have been used in the description above are used only as terms of description and not of limitation. There is no intention of excluding equivalents of the features shown or described.

## Claims

1. An intrastromal corneal insert (102, 108, 114, 116, 124, 130) adapted for introduction into the cornea of a human eye, the insert comprising a physiologically compatible, pliable, synthetic polymeric segment (100,134, 140, 142, 148, 150, 212, 214, 226, 228) having a modulus of elasticity below about 24.1 MPa (3.5 kpsi) and adapted to extend along a circumfereutially extending intrastromal intracomeal channel in said eye and subtend an arc less than 360° of the cornea's circumference when inserted into said intrastromal channel, said segment, alone or in combination with one or more of said segments so inserted, being adapted to effect refractive correction.

2. An insert (134) according to claim 1 that is hollow or tubular.

3. An insert (134) according to claim 1 or claim 2 that, when tubular, further contains a settable polymer, a gel, a drug or a biologically active material.

4. An insert (108, 114) according to claim 1 that has a hexagonal or circular cross section.

5. An insert according to any one of the preceding claims that encircles less than about 320° of the cornea's circumference or encircles less than about 270° of the cornea's circumference.

6. An insert according to any one of the preceding claims wherein said polymeric segment is at least partially coated with an ocular lubricant which, optionally, is hyaluronic acid, methylethylcellulose, dextran solutions, glycerine solutions, polysaccharides, or oligosaccharides and which further optionally contains a drug, biologic or other biologically active material.

7. An insert according to any one of the preceding claims where the polymer of said polymeric segment comprises polyhydroxyethylmethyacrylate (Poly-HEMA), polyvinylpyrrolidone (PVP), polyethylene oxide, or polyacrylates, polyacrylic acid and its derivatives, their copolymers and interpolymers, silicones, cross-linked dextran, cross-linked heparin, or hyaluronic acid

8. An insert according to any one of the claims 1 to 6 where the polymer of said polymeric segment comprises a hydratable polymer which swells upon hydration, a hydratable polymer system which does not swell upon hydration or an elastomer.

9. An insert according to claim 3 where the gel is polyHEMA hydrogel, cross-linked collagen, cross-linked hyaluronic acid, siloxane gels, polyvinyl pyrrolidone or an organic-siloxane gel.

10. An insert according to claim 3 where the drug is dexamethasone, heparin, corticosteroids, anti-nutotics, anti-fibrotics, anti-inflammatory, anti-scar-forming, anti-adhesion, anti-thrombogenic, and anti-angiogenesis factors.

11. An insert according to claim 1 in which the segment contains a reinforcement comprising at least one filament (126) or a woven or matte fabric (132).

12. The insert of claim 1 further comprising at least one other of said segments (302, 306) extending from the first described segment (304) to form an insert precursor (300), a cross-section of one segment increasing stepwise from the other of said segments.

13. The insert of claim 1 further comprising at least one other of said segments (302, 306) extending from the first described segment (304) to form an insert precursor (300), the cross-section of said precursor changing stepwise from segment to segment.

14. The insert of claim 13 wherein said insert precursor (300) comprises three of said segments (302, 304, 306), the cross-section of said precursor increasing from the first segment (302) to the second segment (304) and from the second segment (304) to the third segment (306).

15. A refractive correction system comprising at least two of the segments (148, 150) of claim 1 wherein said segments are of different size.

## Patentansprüche

1. Intrastromaler Kornealeinsatz (102, 108, 114, 116, 124, 130), geeignet für die Einführung in die Kornea eines menschlichen Auges, wobei der Einsatz ein physiologisch kompatibles, biegsames, synthetisches Polymersegment aufweist (100, 134, 140, 142, 148, 150, 212, 214, 226, 228), das ein Elastizitätsmodul unter ca. 24,1 Mpa (3,5 kpsi) aufweist und geeignet ist, sich entlang eines sich umfänglich erstreckenden, intrastromalen, intrakornealen Kanals in dem Auge zu erstrecken und einen Bogen von weniger als 360° Umfangs der Kornea zu unterspannen, wenn es in den intrastromalen Kanal eingesetzt ist, wobei das Segment, allein oder in Kombination mit einem oder mehreren so eingesetzten Segmenten, angepasst ist, eine Brechungskorrektur zu bewirken.

2. Einsatz (134) gemäß Anspruch 1, der hohl oder rohrförmig ist.

3. Einsatz (134) gemäß Anspruch 1 oder 2, der, wenn er rohrförmig ist, ein aushärtbares Polymer, ein Gel, eine Droge oder ein biologisch aktives Material enthält.

4. Einsatz (108, 114) gemäß Anspruch 1, der einen sechsseitigen oder runden Querschnitt aufweist.

5. Einsatz gemäß irgendeinem der vorhergehenden Ansprüche, der weniger als etwa 320° des Umkfangs der Kornea umgibt oder weniger als etwa 270° des Umfangs der Kornea umgibt.

6. Einsatz gemäß irgendeinem der vorhergehenden Ansprüche, wobei das Polymersegment mindestens teilweise mit einem okularen Gleitmittel überzogen ist, das optional Hyaluronsäure, Methylethylzellulose, Dextranlösungen, Glycerinlösungen, Polysaccharide oder Oligosaccharide ist und das ferner optional eine Droge, ein biologisches Präparat oder ein anderes biologisch aktives Material enthält.

7. Einsatz gemäß irgendeinem der vorhergehenden Ansprüche, wobei das Polymer des Polymersegments Polyhydroxyethylmethacrylat (Poly-HEMA), Polyvinylpyrrolidon (PVP), polyethylene Oxide oder Polyacrylate, Polyacrylsäure und ihre Derivate, ihre Kopolymere und Interpolymere, Silikone, querverbundenes Dextran, querverbundenes Heparin oder Hyaluronsäure aufweist.

8. Einsatz gemäß irgendeinem der Ansprüche 1 bis 6, wobei das Polymer des Polymersegments ein hydrierbares Polymer, das bei Hydration anschwillt, ein hydrierbares Polymersystem, das bei Hydration nicht anschwillt, oder ein Elastomer aufweist.

9. Einsatz gemäß Anspruch 3, wobei das Gel ein PolyHEMA-Hydrogel, querverbundenes Kollagen, querverbundene Hyaluronsäure, Siloxangels, Polyvinylpyrrolidone oder ein organisches Siloxangel ist.

10. Einsatz gemäß Anspruch 3, wobei die Droge Dexamethason, Heparin, Corticosteroide, Antimitotika, Antifibrotika, entzündungshemmende, Antinarbenbildungs- , Antiadhäsionsantithrombotische und antiangiogenetische Faktoren aufweist.

11. Einsatz gemäß Anspruch 1, wobei das Segment eine Verstärkung enthält, die mindestens eine Faser (126) oder ein gewobenes oder verfilztes Tuch aufweist.

12. Einsatz gemäß Anspruch 1, ferner aufweisend mindestens ein anderes der Segmente (302, 306), das sich von dem ersten beschriebenen Segment (304) erstreckt, um einen Einsatzwegbereiter (300) zu bilden, wobei ein Querschnitt eines Segments gestuft von dem anderen der Segmente größer wird.

13. Einsatz gemäß Anspruch 1, ferner aufweisend mindestens ein anderes der Segmente (302, 306), das sich von dem ersten beschriebenen Segment (304) erstreckt, um einen Einsatzwegbereiter (300) zu bilden, wobei der Querschnitt des Wegbereiters sich gestuft von Segment zu Segment verändert.

14. Einsatz gemäß Anspruch 13, wobei der Einsatzwegbereiter (300) drei der Segmente (302, 304, 306) aufweist, wobei der Querschnitt des Wegbereiters vom ersten Segment (302) zum zweiten Segment (304) und vom zweiten Segment (304) zum dritten Segment (306) größer wird.

15. Brechungskorrektursystem, das mindestens zwei der Segmente (148, 150) gemäß Anspruch 1 aufweist, wobei die Segmente von unterschiedlicher Größe sind.

## Revendications

1. Insert (102, 108, 114, 116, 124, 130) destiné au stroma cornéen, adapté pour être introduit dans la cornée d'un oeil humain, l'insert comprenant un segment (100, 134, 140, 142, 148, 150, 212, 214, 226, 228) polymérique synthétique, pliable, physiologiquement compatible, ayant un module d'élasticité au-dessous de 24,1 MPa (3,5 kpsi) environ et adapté pour s'étendre le long d'un canal interne au stroma intracornéen s'étendant de manière circonférentielle dans ledit oeil et pour sous-tendre un arc inférieur à 360° de la circonférence de la cornée lors de son introduction dans ledit canal interne au stroma, ledit segment, seul ou en association avec un ou plusieurs desdits segments ainsi introduits, étant adapté pour effectuer une correction de réfraction.

2. Insert (134) selon la revendication 1 qui est creux ou tubulaire.

3. Insert (134) selon la revendication 1 ou 2 qui, s'il est tubulaire, comprend en outre un polymère polymérisable, un gel, un médicament ou une matière biologiquement active.

4. Insert (108, 114) selon la revendication 1 qui présente une section transversale hexagonale ou circulaire.

5. Insert selon l'une quelconque des revendications précédentes qui encercle moins de 320° environ de la circonférence de la cornée ou encercle moins de 270° environ de la circonférence de la cornée.

6. Insert selon l'une quelconque des revendications précédentes, dans lequel ledit segment polymérique est au moins partiellement recouvert d'un lubrifiant oculaire qui est, en option, de l'acide hyaluronique, de la cellulose méthyléthylique, des solutions de dextrane, des solutions de glycérine, des polysaccharides ou des oligosaccharides, et qui comprend en outre de façon optionnelle un médicament, une matière biologique ou une autre matière biologiquement active.

7. Insert selon l'une quelconque des revendications précédentes, dans lequel le polymère dudit segment polymérique comprend du poly-hydroxyéthylméthyacrylate (poly-HEMA), du polyvinylpyrrolidone (PVP), de l'oxyde de polyéthylène, ou des polyacrylates, de l'acide polyacrylique et ses dérivés, leurs copolymères et interpolymères, des silicones, du dextrane réticulé, de l'héparine réticulée, ou de l'acide hyaluronique.

8. Insert selon l'une quelconque des revendications 1 à 6, dans lequel le polymère dudit segment polymérique comprend un polymère pouvant être hydraté, qui gonfle lors de l'hydratation, un système de polymère pouvant être hydraté, qui ne gonfle pas lors de l'hydratation, ou un élastomère.

9. Insert selon la revendication 3, dans lequel le gel est de l'hydrogel polyHEMA, du collagène réticulé, de l'acide hyaluronique réticulé, des gels de siloxane, du pyrrolidone de polyvinyle ou un gel de siloxane organique.

10. Insert selon la revendication 3, dans lequel le médicament est du déxaméthasone, de l'héparine, des corticostéroïdes, des anti- mitotiques, des anti-fibrogènes, des anti-inflammatoires, des agents anti-formation de cicatrices, anti-adhésion, des anti-thrombogènes, et des facteurs anti-angiogenèse.

11. Insert selon la revendication 1 dans lequel le segment contient un renforcement comprenant au moins un filament (126) ou un tissu tissé ou en matte (132).

12. Insert de la revendication 1 comprenant en outre au moins un autre desdits segments (302, 306) s'étendant depuis le premier segment décrit (304) pour former un précurseur d'insert (300), une section transversale d'un segment augmentant par étapes depuis l'autre desdits segments.

13. Insert de la revendication 1 comprenant en outre au moins un autre desdits segments (302, 306) s'étendant depuis le premier segment décrit (304) pour former un précurseur d'insert (300), la section transversale dudit précurseur variant par étapes de segment à segment.

14. Insert de la revendication 13, dans lequel ledit précurseur d'insert (300) comprend trois desdits segments (302, 304, 306), la section transversale dudit précurseur augmentant du premier segment (302) vers le second segment (304) et du second segment (304) vers le troisième segment (306).

15. Système de correction de réfraction comprenant au moins deux des segments (148, 150) de la revendication 1, dans lequel lesdits segments sont de taille différente.
